# EUROPEAN PATENT APPLICATION

(11) **EP 4 289 932 A1**
(43) Date of publication of application: **13.12.2023**
(21) Application number: 22177550.5
(22) Date of filing: 07.06.2022
(51) Int. Cl.: C12M 3/06, C12M 1/00

(54) **CELL CULTIVATION BY USING REMOVABLE TOP-LOADED CHAMBERS IN CELL CULTURE PLATES**

(71) Applicant: Finnadvance Oy, 90220 Oulu (FI)
(72) Inventor: Singh, Prateek, 90220 Oulu (FI); Nguyen, Hoang Tuan, 90220 Oulu (FI)
(74) Representative: Papula Oy

(57) **Abstract**

The present disclosure relates a cell culture plate for microfluidic cell cultivation. The cell culture plate comprises a plurality of cell culture modules arranged adjacent to each other. Each cell culture module comprises two or more culture medium reservoirs connected by two or more flow channels. The flow channels go through a basal chamber arranged under a removable apical chamber. The apical and basal chambers are separated by at least one porous membrane. The basal chamber has a bottom part arranged higher than a bottom part of each of the culture medium reservoirs. In the cell culture plate thus configured, it is possible to replace the apical chambers to grow and study various cell cocultures during the microfluidic cell cultivation. Moreover, cells of the same or different types may be grown on the basal (i.e., bottom) surface of the at least one membrane in each cell culture module under uniform and controlled *in vitro* conditions, without having to invert the cell culture plate. In particular embodiments, a cell culture apparatus and a cell culture incubator both comprising the cell culture plate, as well as a method for cell cultivation by using the cell culture plate are provided.

## Description

### TECHNICAL FIELD

The present disclosure relates generally to the field of biotechnology. In particular, the present disclosure relates to a cell culture plate comprising one or more removable top-loaded chambers, to a cell culture apparatus and a cell culture incubator both using the cell culture plate, as well as to a method for cell cultivation by using the cell culture plate.

### BACKGROUND

Cell culture or cell cultivation involves growing cells of desired type(s) outside a living body under controlled *in vitro* conditions to sustain cell growth and viability. The cell culture is widely used in different biotechnology branches, including cell biology, tissue engineering, biomedical engineering, cell differentiation studies, cell-based biosensors, cell-cell interaction, cell-signaling, cell-migration, physiological and pathophysiological studies, etc.

Environmental conditions created for cultured cells should resemble as closely as possible the conditions experienced by the same cells *in vivo.* This may be done by performing the cell culture in large vessels, such as dishes, spinner and shaker flasks, etc. However, the cell culture conditions provided by these vessels do not truly represent the *in vivo* environment of the cells being cultured. Moreover, since these vessels have a large volume, they consume significant amounts of reagents, culture media, chemicals, etc., thereby making it difficult to control and/or alter the cell culture conditions.

With the advent of microfluidics, new devices and methods configured to cultivate various types of cells, like adherent and non-adherent, under uniform and controlled *in vitro* conditions have been developed. Unlike the conventional cell culture methods based on the above-mentioned vessels, microfluidic cell culture methods may provide continuous nutrient (culture fluid or medium) supply, waste removal, flexibility of schedules, and high automation capability. The less consumption of fluids, their low volumes and therefore the reduced time and cost of cell cultivation make these microfluidic methods particularly interesting for cell-based assays. The main goals of microfluidic cell culture devices are to mimic closely *in vivo* cellular microenvironments and to maintain simplicity for reproducible results.

However, one of the limiting factors of the current microfluidic cell culture devices is the inability to quickly replace top-loaded chambers, so that different cell cocultures may be grown and studied without having to replace the whole cell culture plate in each microfluidic cell culture device.

### SUMMARY

This summary is provided to introduce a selection of concepts in a simplified form that are further described below in the detailed description. This summary is not intended to identify key features of the present disclosure, nor is it intended to be used to limit the scope of the present disclosure.

It is an objective of the present disclosure to provide a technical solution that enables microfluidic cell cultivation by using removable top-loaded (apical) chambers in a cell culture plate.

The objective above is achieved by the features of the independent claims in the appended claims. Further embodiments and examples are apparent from the dependent claims, the detailed description and the accompanying drawings.

According to a first aspect, a cell culture plate is provided. The cell culture plate comprises a housing and a plurality of cell culture modules arranged adjacent to each other in the housing. Each cell culture module of the plurality of cell culture modules comprises at least two culture medium reservoirs, a first chamber, a second chamber, at least one membrane, and at least two flow channels. Each of the at least two culture medium reservoirs has a top part and a bottom part, with the top part having an inlet for a culture medium and the bottom part having an outlet for the culture medium. The first chamber is removably mounted between the at least two culture medium reservoirs such that the first chamber and the at least two culture medium reservoirs are aligned with each other in a first direction. The first chamber is a top-loaded chamber. The second chamber is arranged under the first chamber and aligned with the first chamber in a second direction. The second direction is perpendicular to the first direction. The second chamber has a bottom part having at least two lateral holes. The bottom part of the second chamber is arranged higher than the bottom part of each of the at least two culture medium reservoirs. The at least one membrane has through pores formed therein. The at least one membrane is arranged between the first chamber and the second chamber such that the first chamber and the second chamber are in flow communication with each other via the through pores of the at least one membrane. Each of the at least two flow channels connects the outlet of the bottom part of one of the at least two culture medium reservoirs to one of the at least two lateral holes of the bottom part of the second chamber. In the cell culture plate thus configured, it is possible to replace the first (i.e., top-loaded) chambers to grow and study various cell cocultures during microfluidic cell cultivation, without having to replace the whole cell culture plate. Moreover, in the cell culture plate thus configured, cells of the same or different types may be grown on the basal (i.e., bottom) surface of the at least one membrane in each cell culture module under uniform and controlled *in vitro* conditions, without having to invert the cell culture plate or to use high concentrations of the cells in the culture medium to cause the cells to bond and cover the at least one membrane.

In one exemplary embodiment of the first aspect, the first chamber in at least one cell culture module of the plurality of cell culture modules is shaped as a bottomless hollow tube. With this first chamber, it is possible to deposit cells on the apical (i.e., top) surface of the porous membrane(s). The cells deposited on the apical surface of the porous membrane(s) may be of the same or other type compared to the cells deposited on the basal surface of the porous membrane(s). Thus, the first chamber thus configured may allow one to deposit a cell monolayer on the apical surface of the porous membrane(s) and study the interaction of the cells deposited on the basal and apical surfaces of the porous membrane(s). Moreover, with such configuration, the first chamber is easier and cheaper to manufacture.

In one embodiment of the first aspect, the first chamber in at least one cell culture module of the plurality of cell culture modules is shaped as a hollow tube having a curved or angled bottom. In this embodiment, the curved or angled bottom has at least one cavity and at least one hole formed in each of the at least one cavity. With this first chamber, it is possible to deposit cells on separate portions of the apical surface of the porous membrane(s). This configuration of the first chamber is especially useful for forming similar or different particles under study (e.g., organoids or spheroids) on the apical surface of the porous membrane(s) and study their interaction with the cells deposited on the basal surface of the porous membrane(s).

In one exemplary embodiment of the first aspect, the first chamber in at least one cell culture module of the plurality of cell culture modules is shaped as a hollow tube having a bottom at least partly formed by the at least one membrane. In this case, the replacement of the first chamber will also lead to the replacement of the porous membrane(s) constituting (at least a part of) its bottom. For example, there may be two different first chambers with one having a bottom formed by a fine-pore membrane and another having a bottom formed by a coarse-pore membrane, and a user may use each of these membranes during the microfluidic cell cultivation by properly replacing one first chamber with another.

In one exemplary embodiment of the first aspect, the at least one membrane comprises a first membrane and a second membrane, each of which has through pores formed therein. The first membrane and the second membrane are stacked upon one another. By stacking the porous membranes, it is possible to affect the interaction between the cells deposited on the apical surface of the uppermost porous membrane and the basal surface of the lowermost porous membrane.

In one exemplary embodiment of the first aspect, the first membrane and the second membrane are separated from each other by a spacer (e.g., configured as a ring or a mesh). By using the spacer, it is possible to additionally affect the interaction between the cells deposited on the apical surface of the uppermost porous membrane and the basal surface of the lowermost porous membrane.

In one exemplary embodiment of the first aspect, each of the first membrane and the second membrane has a different thickness and/or a different pore size. By stacking such different membranes, it is possible to form different barriers between the cells deposited on the apical surface of the uppermost porous membrane and the basal surface of the lowermost porous membrane.

According to a second aspect, a cell culture apparatus is provided. The apparatus comprises the cell culture plate according to the first aspect. The apparatus further comprises a flow driving unit configured to cause the culture medium to flow, in each cell culture module of the plurality of cell culture modules, between the at least two culture medium reservoirs via the at least two flow channels and the second chamber. In the apparatus thus configured, it is possible to replace the first (i.e., top-loaded) chambers in the cell culture plate to grow and study various cell cocultures during microfluidic cell cultivation, without having to replace the whole cell culture plate. Moreover, in the apparatus thus configured, cells of the same or different types may be grown on the basal (i.e., bottom) surface of the at least one membrane in each cell culture module under uniform and controlled *in vitro* conditions, without having to invert the cell culture plate or to use high concentrations of the cells in the culture medium to cause the cells to bond and cover the at least one membrane.

According to a third aspect, a cell culture incubator is provided. The cell culture incubator comprises the cell culture apparatus according to the second aspect, and a pipetting station configured to feed the culture medium to each of the at least two culture medium reservoirs in each cell culture module of the plurality of cell culture modules. In the incubator thus configured, it is possible to replace the first (i.e., top-loaded) chambers in the cell culture plate to grow and study various cell cocultures during microfluidic cell cultivation, without having to replace the whole cell culture plate. Moreover, in the incubator thus configured, cells of the same or different types may be grown on the basal (i.e., bottom) surface of the at least one membrane in each cell culture module under uniform and controlled *in vitro* conditions, without having to invert the cell culture plate or to use high concentrations of the cells in the culture medium to cause the cells to bond and cover the at least one membrane.

According to a fourth aspect, a method for cell cultivation by using the cell culture apparatus according to the second aspect is provided. The method starts with the step of providing the culture medium to one of the at least two medium reservoirs in each cell culture module of the plurality of cell culture modules. Then, the method proceeds to the step of causing, by the flow driving unit, the culture medium to flow from said one of the at least two culture medium reservoirs to the rest of the at least two culture medium reservoirs in each cell culture module of the plurality of cell culture modules for a predefined time period. The predefined time period is selected based on the culture medium. By using the cell culture plate thus configured, it is possible to replace the first (i.e., top-loaded) chambers in the cell culture plate to grow and study various cell cocultures during the method, without having to replace the whole cell culture plate. Moreover, it is possible to grow cells of the same or different types on the basal (i.e., bottom) surface of the at least one membrane in each cell culture module under uniform and controlled *in vitro* conditions, without having to invert the cell culture plate or to use high concentrations of the cells in the culture medium to cause the cells to bond and cover the at least one membrane.

In one exemplary embodiment of the fourth aspect, the method further comprises, before the step of providing the cell culture medium to one of the at least two cell culture reservoirs or during the step of causing the culture medium to flow, the step of feeding another culture medium via the first chamber towards the at least one membrane in at least one cell culture module of the plurality of cell culture modules. By so doing, it is possible to cultivate a first type of cells on one side of the at least one membrane and a different second type of cells on another side of the at least one membrane, thereby generating different cell cocultures.

In one exemplary embodiment of the fourth aspect, said another culture medium is a patient-derived tissue culture. By using this type of culture medium, it is possible to create different tissue-based barriers.

In one embodiment of the fourth aspect, the culture medium is caused to flow by applying a positive pressure to the at least two culture medium reservoirs in each cell culture module of the plurality of cell culture modules for the predefined time period. By so doing, it is possible to provide a proper culture medium flow in each cell culture module, thereby improving the cell cultivation in each cell culture module.

In another embodiment of the fourth aspect, the culture medium is caused to flow by applying a positive pressure to said one of the at least two culture medium reservoirs in each cell culture module of the plurality of cell culture modules for the predefined time period, while capping the rest of the at least two culture medium reservoirs. By so doing, it is possible to provide a proper culture medium flow in each cell culture module, thereby improving the cell cultivation in each cell culture module.

In yet another embodiment of the fourth aspect, the at least two culture medium reservoirs in each cell culture module of the plurality of cell culture modules comprises a first culture medium reservoir and a second culture medium reservoir. In this embodiment, the culture medium is caused to flow by:
- applying a first positive pressure to the first culture medium reservoir in each cell culture module of the plurality of cell culture modules for the predefined time interval, while capping the second culture medium reservoir; and
- applying a second positive pressure to the second culture medium reservoir in each cell culture module of the plurality of cell culture modules for the predefined time interval, while capping the first culture medium reservoir.

By so doing, it is possible to provide a proper culture medium flow in each cell culture module, thereby improving the cell cultivation in each cell culture module.

Other features and advantages of the present disclosure will be apparent upon reading the following detailed description and reviewing the accompanying drawings.

### BRIEF DESCRIPTION OF THE DRAWINGS

The present disclosure is explained below with reference to the accompanying drawings in which:
FIG. 1 shows a block diagram of a cell culture plate in accordance with one exemplary embodiment;
FIGs. 2A and 2B show different views of a cell culture module included in the cell culture plate shown in FIG. 1, namely: FIG. 2A shows a top view of the cell culture module, and FIG. 2B shows a sectional side view of the cell culture module (as taken along line A-A in FIG. 2A);
FIG. 3 shows a block diagram of a cell culture apparatus in accordance with one exemplary embodiment;
FIG. 4 shows a block diagram of a cell culture incubator in accordance with one exemplary embodiment; and
FIG. 5 shows a flowchart of a method for cell cultivation by using the cell culture apparatus shown in FIG. 3 in accordance with one exemplary embodiment.

### DETAILED DESCRIPTION

Various embodiments of the present disclosure are further described in more detail with reference to the accompanying drawings. However, the present disclosure may be embodied in many other forms and should not be construed as limited to any certain structure or function discussed in the following description. In contrast, these embodiments are provided to make the description of the present disclosure detailed and complete.

According to the detailed description, it will be apparent to the ones skilled in the art that the scope of the present disclosure encompasses any embodiment thereof, which is disclosed herein, irrespective of whether this embodiment is implemented independently or in concert with any other embodiment of the present disclosure. For example, apparatuses and/or method disclosed herein may be implemented in practice using any numbers of the embodiments provided herein. Furthermore, it should be understood that any embodiment of the present disclosure may be implemented using one or more of the elements presented in the appended claims.

The word "exemplary" is used herein in the meaning of "used as an illustration". Unless otherwise stated, any embodiment described herein as "exemplary" should not be construed as preferable or having an advantage over other embodiments.

Any positioning terminology, such as "left", "right", "top", "bottom", "above", "under", "apical", "basal", etc., may be used herein for convenience to describe one element's or feature's relationship to one or more other elements or features in accordance with the figures. It should be apparent that the positioning terminology is intended to encompass different orientations of the structure and device disclosed herein, in addition to the orientation(s) depicted in the figures. As an example, if one imaginatively rotates the structure or device in the figures 90 degrees clockwise, elements or features described as "top" and "bottom" relative to other elements or features would then be oriented, respectively, "right" and "left" relative to the other elements or features. Therefore, the positioning terminology used herein should not be construed as any limitation of the present disclosure.

Furthermore, although the numerative terminology, such as "first", "second", etc., may be used herein to describe various embodiments, elements or features, it should be understood that these embodiments, elements or features should not be limited by this numerative terminology. This numerative terminology is used herein only to distinguish one embodiment, element or feature from another embodiment, element or feature. For example, a first chamber discussed below could be called a second chamber, and vice versa, without departing from the teachings of the present disclosure.

In the embodiments disclosed herein, a cell may refer to a biological cell, such as a plant cell, an animal cell (e.g., a mammalian cell), a bacterial cell, a fungal cell, or the like. A mammalian cell may be, for example, from a human, a mouse, a horse, a goat, a sheep, a cow, a primate, or the like.

As used in the embodiments disclosed herein, a culture medium, also referred to as a growth medium, may refer to a liquid, suspension, or gel designed to support cellular growth in an artificial environment, i.e., *in vitro.* There are different types of culture media suitable for growing different types of cells. In general, the culture media may be broken into two primary categories: natural media and synthetic media. The natural media are those that are derived from tissue extraction or animal body fluids, such as plasma, lymph, and serum. The synthetic media are those created using a variety of organic and inorganic compounds. Moreover, the culture medium itself may comprise cells, bodies of cells (e.g., organoids), lipid particles, including natural or engineered (e.g., exosomal microvesicles, vacuoles, micelles or lipid particles of various design, virus particles, nanoparticles, etc.).

In the embodiments disclosed herein, a cell culture apparatus may refer to an apparatus having various wells (e.g., reservoirs, chambers, etc.) connected by microchannels in which fluids (i.e., culture media) will exhibit microfluidic behavior in their flow through the microchannels. Such a cell culture apparatus is also referred to as a microfluidic chip in this technical field. The microfluidic cell culture performed by the cell culture apparatus is generally related to cell culture, maintenance and perturbation in micro-scale fluid volumes. The reasons behind the popularity of the microfluidic cell culture are both economic and scientific. The microfluidic chips have the advantages of *in vitro* cell culture (high-throughput, parallel experiments, experiments may be done at the discretion of an experimenter, no need for specialized infrastructure and personnel, etc.) with *in vivo* like performance. For example, in mouse models, drugs are really selected for mice, not humans. By using human cells, drugs are screened for humans. Thus, using humanized microfluidic cell and tissue cultures to screen drug candidates reduces pre-clinical trial time and those drugs entering clinical testing are better suited for humans. This may reduce the probability of adverse effects and increase the chance of showing efficacy resulting in less failures in clinical trials.

In the embodiments disclosed herein, each microchannel of the cell culture apparatus is also referred to as a flow channel and may relate to a sub-millimeter-scale channel that has a hydraulic diameter below 1 mm and is used for fluidically connecting the wells of the cell culture apparatus. In other words, the microchannel or flow channel generally denotes a channel configured to pass different fluids, such, for example, as culture media (e.g., cell suspensions), reagents, or gels, in micro-scale volumes. The microchannel may be shaped such that it has appropriate flow characteristics (e.g., flow rates) depending on particular applications. For example, the microchannel may have a rectangular, e.g., square, or rounded cross-section, as well as be straight, bended, or tilted towards a required direction.

The exemplary embodiments disclosed herein provide a technical solution that enables microfluidic cell cultivation by using removable top-loaded chambers in a cell culture plate. More specifically, the cell culture plate comprises a plurality of cell culture modules arranged adjacent to each other. Each cell culture module comprises two or more culture medium reservoirs connected by two or more flow channels. The flow channels go through a basal chamber arranged under a removable apical (top-loaded) chamber. The apical and basal chambers are separated by at least one porous membrane. The basal chamber has a bottom part arranged higher than a bottom part of each of the culture medium reservoirs. In the cell culture plate thus configured, it is possible to replace the apical chambers to grow and study various cell cocultures during microfluidic cell cultivation, without having to replace the whole cell culture plate. Moreover, in the cell culture plate thus configured, cells of the same or different types may be grown on the basal (i.e., bottom) surface of the at least one membrane in each cell culture module under uniform and controlled *in vitro* conditions, without having to invert the cell culture plate or to use high concentrations of the cells in the culture medium to cause the cells to bond and cover the at least one membrane.

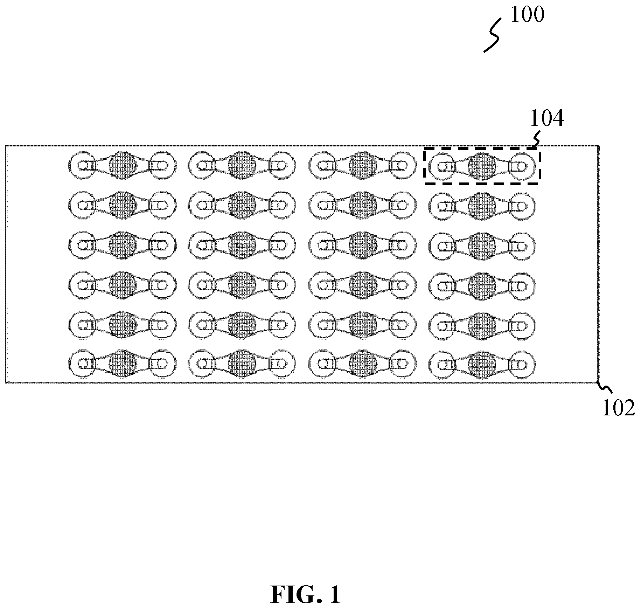

FIG. 1 shows a block diagram of a cell culture plate 100 in accordance with one exemplary embodiment. The cell culture plate 100 is intended for the above-mentioned microfluidic cell culture. As shown in FIG. 1, the cell culture plate 100 comprises a (e.g., rigid) housing 102 and a plurality of cell culture modules 104 arranged adjacent to each other in the housing 102. The cell culture plate 100 may be configured to fit the standard 96, 384, or 1536 microtiter plates. Each cell culture module 104 may be configured as a one- or multi-layered structure made of room-temperature-vulcanizing (RTV) silicone (e.g., RTV615) or polydimethylsiloxane (PDMS), and the cell culture modules may be attached to each other in the order shown in FIG. 1 by using adhesive or plasma bonding to implement the cell culture plate 100. It should be noted that the number, arrangement, and interconnection of the constructive elements constituting the cell culture plate 100, which are shown in FIG. 1, are not intended to be any limitation of the present invention, but merely used to provide a general idea of how the constructive elements may be implemented within the cell culture plate 100.

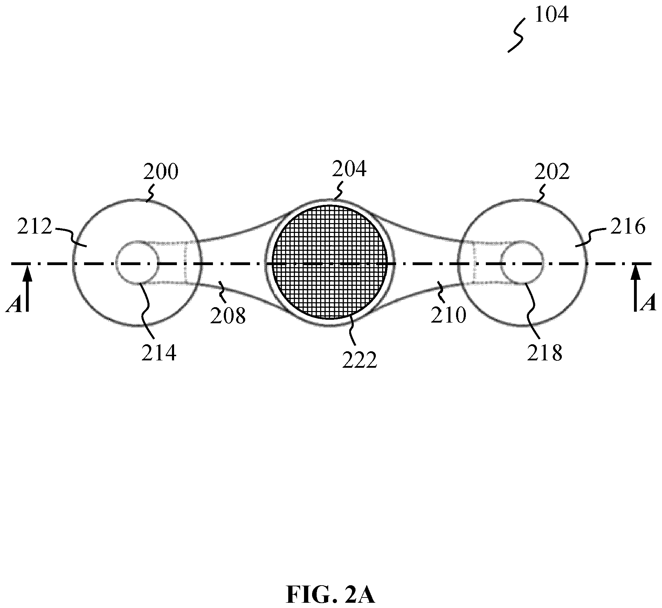

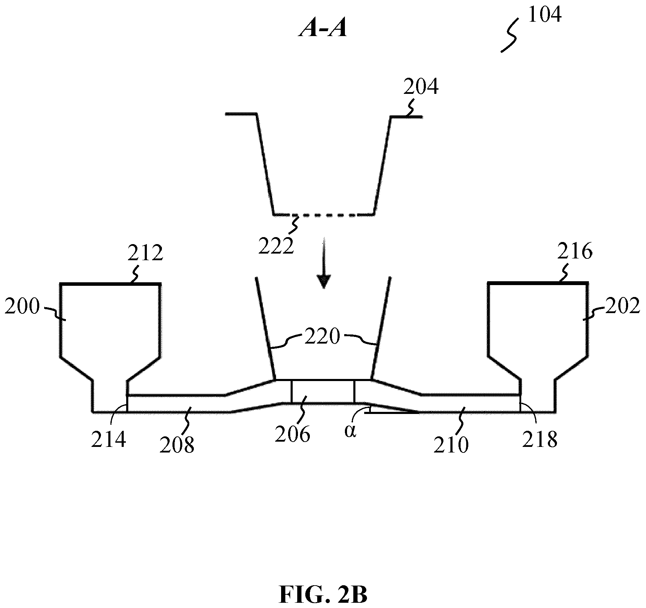

FIGs. 2A and 2B show different views of the cell culture module 104 included in the cell culture plate 100 in accordance with one exemplary embodiment. More specifically, FIG. 2A shows a top view of the cell culture module 104, and FIG. 2B shows a sectional side view of the cell culture module 104 (as taken along line A-A in FIG. 2A). As shown in FIGs. 2A and 2B, the cell culture module 104 comprises a first culture medium reservoir 200, a second culture medium reservoir 202, a first (apical or top) chamber 204, a second (basal or bottom) chamber 206, a first flow channel 208, and a second flow channel 210. It should be again noted that the present disclosure is not limited to the shown number, arrangement, and interconnection of the constructive elements of the cell culture module 104. In some embodiments, there may be more than two medium culture reservoirs, thereby leading to more than two flow channels. In other embodiments, there may be more than two apical chambers and more than two basal chambers between the two culture reservoirs, thereby also increasing the number of the flow channels connecting the two culture reservoirs through the apical chambers and the basal chambers.

The first culture medium reservoir 200 has a top part with an inlet 212 for a culture medium and a bottom part with an outlet 214 for the culture medium. Similarly, the second culture medium reservoir 202 has a top part with an inlet 216 for the culture medium and a bottom part with an outlet 218 for the culture medium. The first and second culture medium reservoirs 200 and 202 may be implemented as identical hollow tubes. Although FIG. 2A shows that each of the first and second culture medium reservoirs 200 and 202 has a circular cross-section, this should not be construed as any limitation of the present disclosure; in some embodiments, any other cross-sectional shapes, such as polygonal, oval, etc., are possible, if required and depending on particular applications. Moreover, the bottom part of each of the first and second culture medium reservoirs 200 and 202 may have a different profile, for example, a positively tapered profile, as shown in FIG. 2B.

The first chamber 204 is a top-loaded (i.e., top-access) chamber that is removably mounted between the first and second medium culture reservoirs 200 and 202 such that the first chamber 204 and the first and second culture medium reservoirs 200 and 202 are aligned with each other in a first (horizontal) direction. In particular, the first chamber 204 may be mounted by lowering it towards the second chamber 206 along guiding or supporting elements 220, as schematically shown by the arrow in FIG. 2. Other ways to mount and secure the first chamber 204 are also possible, such, for example, as different latch and/or spring mechanisms. The first chamber 204 has a bottom (at least partly) formed by a porous membrane 222. When the first chamber 204 is mounted in the cell culture module 104 (i.e., fixed between the guiding or supporting elements 220), the first chamber 204 and the second chamber 206 are in fluid communication with each other via the porous membrane 222. In other embodiments, the porous membrane 222 may be not part of the first chamber 204 itself but be rigidly fixed over and close to the second chamber 206 in the cell culture module 104, so that the replacement of the first chamber 204 will not lead to the replacement of the porous membrane 222 and there will be a fluid communication between the first chamber 204 and the second chamber 206 when the former is mounted in the cell culture module 104. In these embodiments, the first chamber 204 may be shaped as a bottomless hollow tube, for example, with a positively tapered profile (e.g., as shown in FIG. 2B) or with a uniform cross-section. Alternatively, the first chamber 204 may be shaped as a hollow tube having a curved or angled bottom with one or more cavities each having one or more holes. Again, the shown circular cross-section of the first chamber 204 is for illustrative purposes only and should not be considered as any limitation of the present disclosure.

The second chamber 206 is arranged under the (removable) first chamber 204 and aligned with the first chamber 204 in a second (vertical) direction. The second chamber 206 may have a cross-section corresponding to the cross-section of the first chamber 204. The second chamber 206 has a bottom part having two or more lateral holes (not shown in FIGs. 2A and 2B). The second chamber 206 is assumed to have dimensions significantly smaller than the first chamber 204. For example, if the first chamber 204 has a height of about 7 mm, then the height of the second chamber 206 may fall within a range from 50 µm to 150 µm (at height values higher than 150 µm, the second chamber 206 may lose microfluidic properties). Such height values of the first and second chambers 204 and 206 (in addition to the heights of the reservoirs 200 and 202) make the cell culture module 104 more compact, thereby reducing the total dimensions of the cell culture plate 100.

The membrane 222 may be composed of silicone, plastic, protein, natural polymers, artificial polymers (e.g., polyester (PET)), metallic polymers or carbohydrates (e.g., cellulose), and may be formed by using one of the following methods: lithography, stamping, casting, electrospinning or *in situ* polymerization. The through pores of the membrane 222 may have different cross-sectional shapes, such, for example, as triangular, rectangular, square, oval, or polygonal (e.g., hexagonal). The polygonal cross-section shape of the through pores may refer to a convex or concave polygon. In some other embodiments, the through pores may have different cross-sectional shapes, such as any combination of two or more of the above-mentioned cross-sectional shapes (e.g., the combination of circular and square cross-section shapes). Furthermore, each of the through pores may have a pore size varying within a predefined range of values. For example, the pore size may vary from 0.2 µm to 10 µm. In general, the predefined range of values for the pore size (as well as the spacing between the through pores) is selected based on certain cells to be deposited on the membrane 222. More specially, the pore sizes may be more or less than sizes of the cells to be deposited on the membrane 222. For example, if the pore size is less than the cell size, then it is possible to prevent the cells from migrating between the basal and apical surfaces of the membrane 222.

The first and second flow channels 208 and 210 are microchannels providing the passage of the culture medium between the first and second culture medium reservoirs 200 and 202 through the second chamber 206. In particular, the first flow channel 208 connects the outlet 214 of the bottom part of the first culture medium reservoir 200 to one or more of the lateral holes arranged on the left side of the bottom part of the second chamber 206. The second flow channel 210 connects the outlet 218 of the bottom part of the second culture medium reservoir 202 to one or more of the lateral holes arranged on the right side of the bottom part of the second chamber 206. Each of the first and second flow channels 208 and 210 may have a longitudinal section and a cross-section which allow one to achieve required flow characteristics (e.g., an appropriate flow rate). For example, each of the first and second flow channels 208 and 210 may a variable channel height and a variable channel width which increase (e.g., gradually) towards the second chamber 206.

As also follows from FIG. 2B, each of the first flow channel 208 and the second flow channel 210 is at least partly bended or tilted relative to the first (horizontal) direction such that the bottom part of the second chamber 206 is arranged higher than the bottom part of the first/second culture medium reservoir 200/202 (i.e., higher than the end of the outlet 214/218). More specifically, each of the first flow channel 208 and the second flow channel 210 has a tilting angle a relative to the first (horizontal) direction, which is preferably within a range of 5 degrees to 45 degrees to provide appropriate flow characteristics.

In some other embodiments, the cell culture module 104 may comprise, instead of the single membrane 222, two or more porous membranes either attached to the bottom of the first chamber 204 or rigidly fixed over and close to the second chamber 222. For example, if there are two porous membranes used in the cell culture module 104, they may be either arranged adjacent to each other in one plane (e.g., at the bottom of the first chamber 204) or be stacked upon one another (e.g., over and close to the second chamber 206). Moreover, these two or more membranes may be separated from each other by a spacer. The spacer may be implemented as a ring or a mesh made of one of the following materials: plastic (polypropylene (PP), polystyrene (PS), polycarbonate (PC), and others), silicone, metal, ceramic, and hydrogel. The spacer material may also disintegrate over time. Finally, the spacer material may be the same as used for the membrane 222 or the cell culture module 104. It is also possible that each of these two or more membranes has a different thickness and/or a different pore size to form different barriers between the cells on both (top and bottom) sides of such a multi-membrane structure.

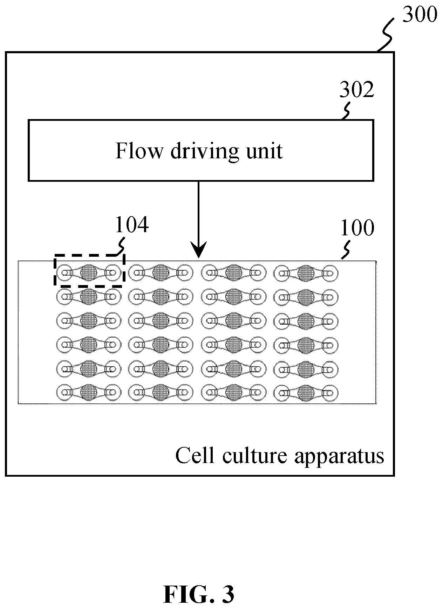

FIG. 3 shows a block diagram of a cell culture apparatus 300 in accordance with one exemplary embodiment. The apparatus 300 is intended for the above-mentioned microfluidic cell culture. As shown in FIG. 3, the apparatus 300 comprises the cell culture plate 100 and a flow driving unit 302 coupled to the cell culture plate 100. The flow driving unit 302 is configured to cause the culture medium to flow, in each cell culture module 104 of the cell culture plate 100, between the first and second culture medium reservoirs 200 and 202 through the second chamber 206. In one embodiment, the flow driving unit 302 may be a rocking platform that is configured to periodically rock the cell culture plate 100, thereby providing the culture medium flow. In another embodiment, the flow driving unit 302 may be a pneumatic pump that is configured to supply a compressed gas or a pressurized air to the inlet of each of the first and second culture medium reservoirs 200 and 202 in each cell culture module 104 of the cell culture plate 100, thereby causing the culture medium flow therein. In yet another embodiment, the flow driving unit 302 may be configured to cause the culture medium flow in each cell culture module 104 of the cell culture plate 100 by pipetting the culture medium from the first culture medium reservoir 200 to the second culture medium reservoir 202, or vice versa, at regular time intervals or based on a level of the culture medium in each of the reservoirs. It should be noted that the number, arrangement, and interconnection of the constructive elements constituting the apparatus 300, which are shown in FIG. 3, are not intended to be any limitation of the present invention, but merely used to provide a general idea of how the constructive elements may be implemented within the apparatus 300. For example, the cell culture plate 100 may be replaced with two or more similar cell culture plates, and the flow driving unit 302 may be replaced with two or more flow driving units each configured to control a culture medium flow in one of the cell culture plates.

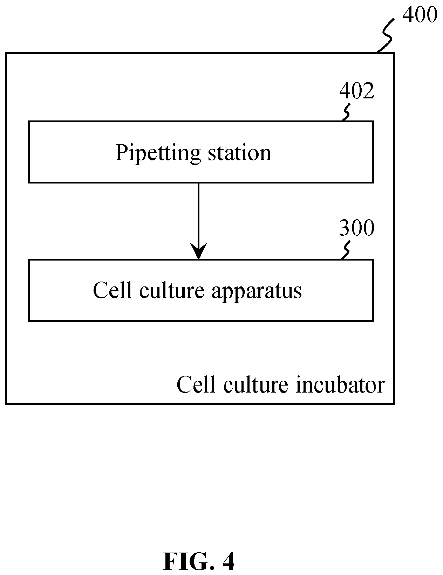

FIG. 4 shows a block diagram of a cell culture incubator 400 in accordance with one exemplary embodiment. The cell culture incubator 400 comprises the cell culture apparatus 300, and a pipetting station 402 coupled to the apparatus 300. More specifically, the pipetting station 402 is configured to feed the culture medium to each of the first and second culture medium reservoirs 200 and 202 in each cell culture module 104 of the cell culture plate 100. The pipetting station 402 is well-known in this technical field, for which reason its description is omitted herein.

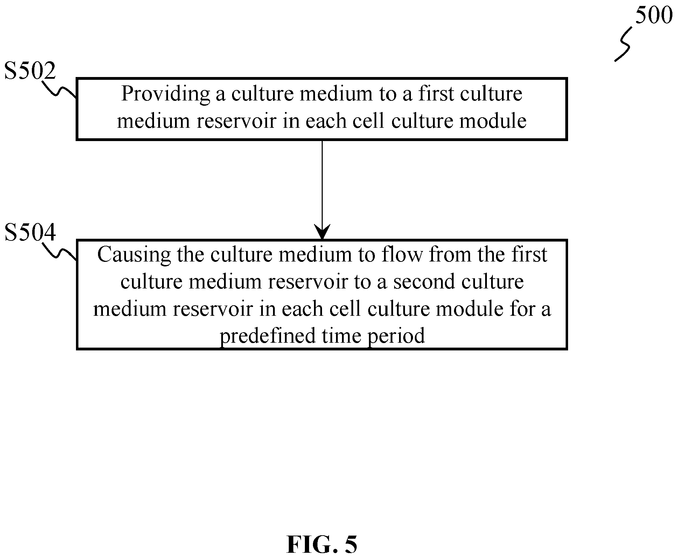

FIG. 5 shows a flowchart of a method 500 for cell cultivation by using the cell culture apparatus 300 in accordance with one exemplary embodiment. The method 500 starts with a step S502, in which a required culture medium is provided, e.g., the pipetting station 402, to one of the first and second culture medium reservoirs 200 and 202 in each cell culture module 104 of the cell culture plate 100. Let us assume that the culture medium is provided to the first culture medium reservoir 200. Then, the method 500 proceeds to a step S504, in which the flow driving unit 302 causes the culture medium to flow from the first culture medium reservoir 200 to the second culture medium reservoir 202 in each cell culture module 104 of the cell culture plate 100 for a predefined time period. The predefined time period may be selected based on the culture medium (i.e., a type of cells used therein). Assuming that each cell culture module 104 comprises only one membrane 222, the cell deposition on the basal surface of the membrane 222 may be achieved if a flow rate is greater than gravity and a continuous flow lasts at least 30 min, preferably 60 min.

In one embodiment, the method 500 may comprise, before the step S502 or during the step S504, an additional step, in which a different culture medium is fed, e.g., by the pipetting station 402, via the first chamber 204 towards the membrane 222 in one or more cell culture modules 104 of the cell culture plate 100. If the first chamber 204 is shaped as a bottomless hollow tube, a cell monolayer is formed on the apical surface of the membrane 222, and this cell monolayer interacts with a cell layer forming on the basal surface of the membrane 222 via the through pores of the membrane 222. If the first chamber 204 is shaped as a hollow tube having an angled or curved bottom with multiple "holey" microcavities, these microcavities are bonded to the membrane 222, for which reason cells from the different culture medium are trapped to form a single organoid and/or spheroid in each microcavity. In this case, the flow channels 208 and 210 may perfuse the culture medium through the membrane 222 to keep the organoids and/or spheroids alive. If the first chamber 204 is implemented as shown in FIG. 2B (i.e., has its bottom at least partly formed by the membrane 222), it is possible to demount the first chamber 204 from the cell culture module 104 first and then to place the different cell culture (e.g., a patient-derived tissue culture) on the apical surface of the membrane 222 by using, for example, a tweezer, whereupon the first chamber 204 may be again mounted in the cell culture module 104. Irrespective of its implementation, the first chamber 204 may be repeatedly replaced during the method 500, if required and depending on particular applications.

In one embodiment, the step S504 of the method 500 may be performed by applying a positive pressure to each of the first culture medium reservoir 200 and the second culture medium reservoir 202 in each cell culture module 104 of the cell culture plate 100 for the predefined time period (e.g., 60 min). In another embodiment, the step S504 of the method 500 may be performed by applying a positive pressure to the first two culture medium reservoir 200 in each cell culture module 104 of the cell culture plate 100 for the predefined time period (e.g., 60 min), while capping the second culture medium reservoir 202, or vice versa. In yet another embodiment, the step S504 of the method 500 may be performed by: (i) applying a first positive pressure to the first culture medium reservoir 200 in each cell culture module 104 of the cell culture plate 100 for the predefined time interval, while capping the second culture medium reservoir 202; and (ii) applying a second positive pressure to the second culture medium reservoir 202 in each cell culture module 104 of the cell culture plate 100 for the predefined time interval, while capping the first culture medium reservoir 200. The selection of each of the above-indicated embodiments of the step S504 depends on particular applications.

Although the exemplary embodiments of the present disclosure are described herein, it should be noted that any various changes and modifications could be made in the embodiments of the present disclosure, without departing from the scope of legal protection which is defined by the appended claims. In the appended claims, the word "comprising" does not exclude other elements, steps or operations, and the indefinite article "a" or "an" does not exclude a plurality. The mere fact that certain measures are recited in mutually different dependent claims does not indicate that a combination of these measures cannot be used to advantage.

## Claims

1. A cell culture plate comprising:
a housing; and
a plurality of cell culture modules arranged adjacent to each other in the housing, each cell culture module of the plurality of cell culture modules comprising:
at least two culture medium reservoirs each having a top part and a bottom part, the top part having an inlet for a culture medium and the bottom part having an outlet for the culture medium;
a first chamber removably mounted between the at least two culture medium reservoirs such that the first chamber and the at least two culture medium reservoirs are aligned with each other in a first direction, the first chamber being a top-loaded chamber;
a second chamber arranged under the first chamber and aligned with the first chamber in a second direction, the second direction being perpendicular to the first direction, the second chamber having a bottom part having at least two lateral holes, the bottom part of the second chamber being arranged higher than the bottom part of each of the at least two culture medium reservoirs;
at least one membrane having through pores formed therein, the at least one membrane being arranged between the first chamber and the second chamber such that the first chamber and the second chamber are in flow communication with each other via the through pores of the at least one membrane; and
at least two flow channels each connecting the outlet of the bottom part of one of the at least two culture medium reservoirs to one of the at least two lateral holes of the bottom part of the second chamber.

2. The cell culture plate of claim 1, wherein the first chamber in at least one cell culture module of the plurality of cell culture modules is shaped as a bottomless hollow tube.

3. The cell culture plate of claim 1, wherein the first chamber in at least one cell culture module of the plurality of cell culture modules is shaped as a hollow tube having a curved or angled bottom, the curved or angled bottom having at least one cavity and at least one hole formed in each of the at least one cavity.

4. The cell culture plate of claim 1, wherein the first chamber in at least one cell culture module of the plurality of cell culture modules is shaped as a hollow tube having a bottom at least partly formed by the at least one membrane.

5. The cell culture plate of any one of claims 1 to 4, wherein the at least one membrane comprises a first membrane and a second membrane, each of the first membrane and the second membrane having through pores formed therein, and the first membrane and the second membrane being stacked upon one another.

6. The cell culture plate of any one of claim 5, wherein the first membrane and the second membrane are separated from each other by a spacer.

7. The cell culture plate of claim 5 or 6, wherein each of the first membrane and the second membrane has a different thickness and/or a different pore size.

8. A cell culture apparatus comprising:
the cell culture plate according to any one of claims 1 to 7; and
a flow driving unit configured to cause the culture medium to flow, in each cell culture module of the plurality of cell culture modules, between the at least two culture medium reservoirs via the at least two flow channels and the second chamber.

9. A cell culture incubator comprising:
the cell culture apparatus according to claim 8; and
a pipetting station configured to feed the culture medium to each of the at least two culture medium reservoirs in each cell culture module of the plurality of cell culture modules.

10. A method for cell cultivation by using the cell culture apparatus according to claim 8, the method comprising:
(a) providing the culture medium to one of the at least two medium reservoirs in each cell culture module of the plurality of cell culture modules; and
(b) causing, by the flow driving unit, the culture medium to flow from said one of the at least two culture medium reservoirs to the rest of the at least two culture medium reservoirs in each cell culture module of the plurality of cell culture modules for a predefined time period, the predefined time period being selected based on the culture medium.

11. The method of claim 10, further comprising, before step (a) or during step (b), feeding another culture medium via the first chamber towards the at least one membrane in at least one cell culture module of the plurality of cell culture modules.

12. The method of claim 11, wherein said another culture medium is a patient-derived tissue culture.

13. The method of any one of claims 10 to 12, wherein step (b) comprises applying a positive pressure to the at least two culture medium reservoirs in each cell culture module of the plurality of cell culture modules for the predefined time period.

14. The method of any one of claims 10 to 12, wherein step (b) comprises applying a positive pressure to said one of the at least two culture medium reservoirs in each cell culture module of the plurality of cell culture modules for the predefined time period, while capping the rest of the at least two culture medium reservoirs.

15. The method of any one of claims 10 to 12, wherein the at least two culture medium reservoirs in each cell culture module of the plurality of cell culture modules comprises a first culture medium reservoir and a second culture medium reservoir, and wherein step (b) comprises:
- applying a first positive pressure to the first culture medium reservoir in each cell culture module of the plurality of cell culture modules for the predefined time interval, while capping the second culture medium reservoir; and
- applying a second positive pressure to the second culture medium reservoir in each cell culture module of the plurality of cell culture modules for the predefined time interval, while capping the first culture medium reservoir.
